# EUROPEAN PATENT SPECIFICATION

(11) **EP 4 440 512 B1**
(45) Date of publication and mention of the grant of the patent: **12.08.2026**
(21) Application number: 22826266.3
(22) Date of filing: 16.11.2022
(51) Int. Cl.: A61F 5/443, A61B 50/30, A61F 5/445

(54) **ASYMMETRIC ADHESIVE PACKAGING ENCLOSURE FOR EASE OF MATERIAL REMOVAL**
ASYMMETRISCH KLEBENDE VERPACKUNG FÜR ERLEICHTERTE MATERIALENTNAHME
DISPOSITIF D'EMBALLAGE AVEC ADHESION ASYMMÉTRIQUE POUR UN ENLÈVEMENT DE MATÉRIEL FACILITÉ

(30) Priority: 01.12.2021 US 202163284682 P
(43) Date of publication of application: 09.10.2024
(73) Proprietor: Hollister Incorporated, Libertyville, IL 60048 (US)
(72) Inventor: DEFANTE, Adrian P., Libertyville, IL 60048 (US); JOCKEL, Mark W., Libertyville, IL 60048 (US)
(74) Representative: FRKelly
(86) International application number: PCT/US2022/050041
(87) International publication number: WO 2023/101813

(56) References cited:
- EP-A2- 1 938 951
- US-A1- 2014 163 496
- US-A1- 2016 193 003
- US-A1- 2018 177 626

## Description

### BACKGROUND

The following description relates generally to a packaging enclosure and more particularly to a packaging enclosure for an adhesive ostomy appliance.

Adhesive appliances, such as barrier rings are packaged and stored in enclosures to maintain the cleanliness and integrity of the appliance. Barrier rings are formed from a material that is, to some extent, inherently adhesive or sticky. A typical enclosure uses a molded clamshell or clamshell-like enclosure or package to secure and maintain the appliance (barrier ring). The barrier ring has release liners, which are protective coverings, on one or both sides of the barrier ring as it is stored in the enclosure.

To use the barrier ring, the enclosure is opened and the barrier ring is removed from the enclosure. The release liners are removed, and the barrier ring is stretched or otherwise contoured to the desired shape.

While current package configurations function well to protect the barriers in the enclosure, one issue with such enclosures is that a user has to remove the barrier from the enclosure and then remove the release liners from one or both sides of the barrier prior to use. This can be problematic in an ostomy care setting in which a user is attempting to remove and apply the barrier to the area around the user's stoma.

EP1938951A2 discloses a container (30) having a plastic lid (34) and a metal tray (34) which are sealed together to form the container (30). The plastic lid (34) is preferably domed and has a sealing layer co-extruded, coated or laminated to it to bond the lid (34) and tray (32).

US2014163496A1 discloses a package for an ostomy sealing member for sealing around a stoma in relation to an ostomy appliance, said sealing member comprising a hypoallergenic, mouldable adhesive material, said sealing member having an annular periphery and an opening therein for receiving a stoma, wherein the opening is non-concentric with the periphery.

US2016193003A1 discloses a package for an ostomy barrier ring. The package includes a first shell, a second shell and a hinge connecting the first and second shells. The first shell includes a first stacking wall having a first interior side and a first exterior side, a first nesting structure formed on the first stacking wall, and a first sidewall extending from and about the first stacking wall. The second shell includes a second stacking wall having a second interior side and a second exterior side, a second nesting structure formed on the second stacking wall, and a second sidewall extending from and about the second stacking wall. The hinge allows the first shell and second shell to rotate relative to one another. A plurality of packages may be stacked with the first nesting structure of one package received in the second nesting structure of an adjacent package.

US2018177626A1 discloses an assembly (100) for an ostomy device (104) comprising a package (102). The package (102) comprises at least a first and a second package portion (106, 108) which are interconnected in such a way that the package (102) is in a closed state when the first and second package portions (106, 108) are in a first mutual position and such that the package (102) is in an open state when the first and second package portions (106, 108) are in a second mutual position. The assembly further comprises a release liner (110) which at least partially covers the adhesive front surface of the ostomy device (104) at least in the closed state of the package. At least a portion of the release liner (110) is attached to the package (102) or formed integrally therewith.

Accordingly, it is desirable to provide an ostomy barrier ring package that permits readily removing the appliance in a ready to use state. Desirably, such a package reduces the handling needed by the user in preparing and applying the barrier ring.

### SUMMARY

The present disclosure provides an ostomy appliance and packaging enclosure as detailed in claim 1. Advantageous features are provided in dependent claims.

In an aspect, a packaging enclosure for an ostomy appliance such as a barrier ring that is adhesive, includes a body having a base and an upstanding sidewall integral and contiguous with the base. The upstanding side wall has a peripheral edge opposite the base. The base and sidewall define a storage region for the barrier ring. The base and sidewall are formed from a first material.

A cover element extends over storage region on the sidewall peripheral edge. The cover element is formed from a second material different from the first material. The second material has a higher affinity for the ostomy appliance, e.g., the barrier ring, than the first material affinity for the ostomy appliance. In this manner, as the cover is removed from the body, the ostomy appliance, e.g., the barrier ring, remains affixed to the cover.

In an embodiment, the body further includes a pedestal formed therein that extends upwardly from the base. In embodiments, the cover element is sealed to the sidewall peripheral edge. The sidewall can be circular in cross-section.

In an embodiment, to further facilitate use, the cover element can include a grasping region that extends from an edge thereof.

In an embodiment, the body can be formed of a polymeric material such as polyethylene (PE), polyethylene terephthalate (PET), polypropylene, polyester, polyimide, polyoxymethylene, organosiloxane, or cellulosic or paper-based materials or combinations thereof. The cover likewise can be formed of a polymeric material, such as polyethylene (PE), polyethylene terephthalate (PET), polypropylene, polyester, polyimide, polyoxymethylene, organosiloxane, or cellulosic or paper-based materials or combinations thereof. Other suitable materials will be recognized by those skilled in the art.

In another aspect, an ostomy appliance and packaging enclosure includes an ostomy appliance, such as a barrier ring, which appliance has first and second surfaces. The ostomy appliance may be formed from an adhesive material.

In embodiments, a packaging enclosure includes a body having a base and an upstanding sidewall integral and contiguous with the base. The body has a peripheral edge opposite the base. The base and sidewall define a storage region for the appliance. The base and sidewall formed from a first material.

A cover element is formed form a second material different from the first material. The ostomy appliance is positioned in the storage region with the first surface positioned on the base and the cover element is positioned on the upstanding sidewall peripheral wall and in contact with the ostomy appliance second surface. The second material has a higher affinity for the ostomy appliance than the first material affinity for the ostomy appliance, such that when the cover is removed from the body, the ostomy appliance, e.g., the barrier ring, remains affixed to the cover. The cover can be sealed to the upstanding side wall.

In embodiments, the barrier can be formed from a hydrocolloid material. The barrier ring material can further include ceramide. The packaging enclosure body can be formed of a polymeric material, such as polyethylene (PE), polyethylene terephthalate (PET), polypropylene, polyester, polyimide, polyoxymethylene, organosiloxane, or cellulosic or paper-based materials or combinations thereof. The cover likewise can be formed of a polymeric material, such as polyethylene (PE), polyethylene terephthalate (PET), polypropylene, polyester, polyimide, polyoxymethylene, organosiloxane, or cellulosic or paper-based materials or combinations thereof. Other suitable materials will be recognized by those skilled in the art.

Other objects, features, and advantages of the disclosure will be apparent from the following description, taken in conjunction with the accompanying sheets of drawings, wherein like numerals refer to like parts, elements, components, steps, and processes.

### BRIEF DESCRIPTION OF THE DRAWINGS

FIG. 1 is a perspective view of an example of an ostomy barrier ring;
FIG. 2 is a side view of a packaging enclosure with a cover according to an embodiment;
FIG. 3 illustrates the barrier ring of FIG. 1 in the packaging enclosure of FIG. 2;
FIG. 4 shows the barrier ring of FIG. 3 as it is being removed from the enclosure;
FIG. 5 illustrates the barrier ring removed from the enclosure with the packaging enclosure cover thereon; and
FIG. 6 shows the enclosure with the barrier ring removed.

### DETAILED DESCRIPTION

While the present disclosure is susceptible of embodiment in various forms, there is shown in the drawings and will hereinafter be described one or more embodiments with the understanding that the present disclosure is to be considered illustrative only and is not intended to limit the disclosure to any specific embodiment described or illustrated.

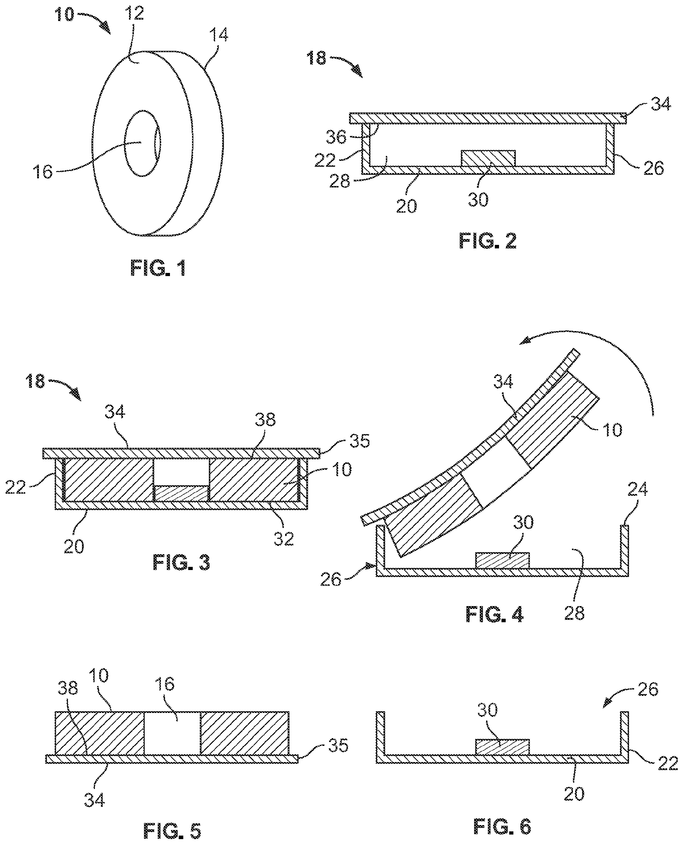

FIG. 1 illustrates an ostomy appliance, such as a barrier ring 10. The barrier ring 10 may be formed from a pressure sensitive adhesive, such as a flexible hydrocolloid material, that may include, for example, ceramide. The barrier ring 10 may have first and second sides 12, 14 and an open central region 16. Referring to FIGS. 2-4, the barrier ring 10 may be packaged and stored in a packaging enclosure 18. In an embodiment, the enclosure 18 may include a base 20 and an upstanding sidewall 22 having a peripheral edge 24. The base 20 and sidewall 22 may define a cup-shaped body 26 having an interior or storage region 28 in which the barrier 10 may be positioned. An upstanding pedestal 30 can be formed in the base 20, such that the open central region 16 of the barrier ring 10 is centered about the pedestal 30. The barrier ring 10 may be positioned in the enclosure 18 with the first side 12 positioned on the base 20 to define a first interface 32.

A cover element 34 may have a barrier ring facing surface 36 that is positioned on the second side 14 of the barrier ring 10 to define a second interface 38. The cover element 34 may extend over the sidewall peripheral edge 24. In embodiments, the cover 34 may be sealed to the peripheral edge 24 to close or seal the enclosure 18. The cover 34 can be formed from a flexible, pliable material, such as a film, that permits the cover 34 to be readily peeled off of the enclosure 18. In embodiments, a portion of an edge of the cover 34 may extend over and beyond the peripheral edge 24 to form a grasping region 35.

To facilitate removal of the barrier ring 10 from the enclosure 18, the materials of the enclosure 18 and the cover 34 may be such that the cover 34 has a higher affinity for the barrier ring 10 material than the barrier ring 10 material has for the enclosure 18 material. That is, the barrier ring 10 may have a greater adherence to the cover 34 than to the enclosure 18. Thus, with the barrier ring 10 stored in the package 18, a user may pull the cover 34 from the enclosure 18, the barrier ring 10 releases from the enclosure 18 and remains affixed to the cover 34. That is, the barrier ring 10 may release from the enclosure 18 and pull away with the cover 34. The user can then readily remove the barrier ring 10 from the cover 34 to use the ring 10.

One barrier 10 material may be a flexible hydrocolloid material, that may include, for example, ceramide. Accordingly, in an embodiment, the body 26 can be formed from a polymeric material, such as polyethylene (PE), polyethylene terephthalate (PET), polypropylene, polyester, polyimide, polyoxymethylene, organosiloxane, or cellulosic or paper-based materials or combinations thereof. Other suitable materials will be recognized by those skilled in the art. The cover 34 can likewise be formed from a polymeric material, such as polyethylene (PE), polyethylene terephthalate (PET), polypropylene, polyester, polyimide, polyoxymethylene, organosiloxane, or cellulosic or paper-based materials or combinations thereof. Other suitable materials will be recognized by those skilled in the art. In such a configuration, the cover 34 may have a greater affinity for the barrier ring 10 than the body 26 affinity for the barrier ring 10. As such, as the cover 34 is removed from the body 26, the barrier ring 10 may remain adhered to the cover 34 for ease of handling and use by a user.

In the present disclosure, the words "a" or "an" are to be taken to include both the singular and the plural. Conversely, any reference to plural items shall, where appropriate, include the singular. In additions, various features described with respect to any of the embodiments above may be used together, implemented in, or replace features in any of the other embodiments described above.

From the foregoing it will be observed that numerous modifications and variations can be effectuated without departing from the scope of the novel concepts of the present invention. It is to be understood that no limitation with respect to the specific embodiments illustrated is intended or should be inferred. The disclosure is intended to cover by the appended claims all such modifications as fall within the scope of the claims.

## Claims

1. An ostomy appliance (10) and packaging enclosure (18), comprising:
an ostomy appliance (10), the ostomy appliance having first and second surfaces (12, 14), the ostomy appliance formed from an adhesive material;
a packaging enclosure (18) comprising:
a body having a base (20) and an upstanding sidewall (22) integral and contiguous with the base and having a peripheral edge (24) opposite the base, the base and sidewall defining a storage region (28), the base and sidewall formed from a first material; and
a cover element (34),
wherein the ostomy appliance (10) is positioned in the storage region with the first surface (12) positioned on the base,
wherein the cover element (34) is positioned on the upstanding sidewall and in contact with the ostomy appliance second surface (14), and
**characterised in that** the cover element (34) is formed from a second material, the second material having a higher affinity for the ostomy appliance (10) than the first material affinity for the ostomy appliance.

2. The ostomy appliance (10) and packaging enclosure (18) of claim 1, wherein the ostomy appliance (10) remains affixed to the cover (34) when the cover is removed from the packaging enclosure.

3. The ostomy appliance (10) and packaging enclosure (18) of claim 1, wherein the cover (34) is sealed to the upstanding side wall (22).

4. The ostomy appliance (10) and packaging enclosure (18) of claim 1, wherein the body is formed of polyethylene (PE), polyethylene terephthalate (PET), polypropylene, polyester, polyimide, polyoxymethylene, organosiloxane, or cellulosic or paper-based materials or combinations thereof.

5. The ostomy appliance (10) and packaging enclosure (18) of claim 1, wherein the cover (34) is formed of polyethylene (PE), polyethylene terephthalate (PET), polypropylene, polyester, polyimide, polyoxymethylene, organosiloxane, or cellulosic or paper-based materials or combinations thereof.

6. The ostomy appliance (10) and packaging enclosure (18) of claim 4, wherein the cover (34) is formed of polyethylene (PE), polyethylene terephthalate (PET), polypropylene, polyester, polyimide, polyoxymethylene, organosiloxane, or cellulosic or paper-based materials or combinations thereof.

7. The ostomy appliance (10) and packaging enclosure (18) of claim 1, wherein the ostomy appliance is a barrier ring (10).

8. The ostomy appliance (10) and packaging enclosure (18) of claim 7, wherein the barrier ring (10) is formed from a hydrocolloid material.

9. The ostomy appliance (10) and packaging enclosure (18) of claim 8, wherein the barrier ring material further includes ceramide.

## Patentansprüche

1. Ostomievorrichtung (10) und Verpackung (18), umfassend:
eine Ostomievorrichtung (10), wobei die Ostomievorrichtung eine erste und eine zweite Oberfläche (12, 14) aufweist und die Ostomievorrichtung aus einem Klebematerial gebildet ist;
eine Verpackung (18), umfassend:
einen Körper, aufweisend einen Boden (20) und eine aufrechte Seitenwand (22), die einstückig und zusammenhängend mit dem Boden ist und eine dem Boden gegenüberliegende Umfangskante (24) aufweist, wobei der Boden und die Seitenwand einen Aufbewahrungsbereich (28) definieren, wobei der Boden und die Seitenwand aus einem ersten Material gebildet sind; und
ein Abdeckelement (34),
wobei die Ostomievorrichtung (10) in dem Aufbewahrungsbereich angeordnet ist, wobei die erste Oberfläche (12) auf dem Boden angeordnet ist,
wobei das Abdeckelement (34) an der aufrechten Seitenwand angeordnet ist und mit der zweiten Oberfläche (14) der Ostomievorrichtung in Kontakt steht, und
**dadurch gekennzeichnet, dass** das Abdeckelement (34) aus einem zweiten Material gebildet ist, wobei das zweite Material eine höhere Affinität für die Ostomievorrichtung (10) aufweist als die Affinität des ersten Materials für die Ostomievorrichtung.

2. Ostomievorrichtung (10) und Verpackung (18) nach Anspruch 1, wobei die Ostomievorrichtung (10) an der Abdeckung (34) befestigt bleibt, wenn die Abdeckung von der Verpackung entfernt wird.

3. Ostomievorrichtung (10) und Verpackung (18) nach Anspruch 1, wobei die Abdeckung (34) mit der aufrechten Seitenwand (22) versiegelt ist.

4. Ostomievorrichtung (10) und Verpackung (18) nach Anspruch 1, wobei der Körper aus Polyethylen (PE), Polyethylenterephthalat (PET), Polypropylen, Polyester, Polyimid, Polyoxymethylen, Organosiloxan oder zellulosehaltigen oder papierbasierten Materialien oder Kombinationen davon gebildet ist.

5. Ostomievorrichtung (10) und Verpackung (18) nach Anspruch 1, wobei die Abdeckung (34) aus Polyethylen (PE), Polyethylenterephthalat (PET), Polypropylen, Polyester, Polyimid, Polyoxymethylen, Organosiloxan oder zellulosehaltigen oder papierbasierten Materialien oder Kombinationen davon gebildet ist.

6. Ostomievorrichtung (10) und Verpackung (18) nach Anspruch 4, wobei die Abdeckung (34) aus Polyethylen (PE), Polyethylenterephthalat (PET), Polypropylen, Polyester, Polyimid, Polyoxymethylen, Organosiloxan oder zellulosehaltigen oder papierbasierten Materialien oder Kombinationen davon gebildet ist.

7. Ostomievorrichtung (10) und Verpackung (18) nach Anspruch 1, wobei die Ostomievorrichtung ein Barrierering (10) ist.

8. Ostomievorrichtung (10) und Verpackung (18) nach Anspruch 7, wobei der Barrierering (10) aus einem Hydrokolloidmaterial gebildet ist.

9. Ostomievorrichtung (10) und Verpackung (18) nach Anspruch 8, wobei das Barriereringmaterial ferner Ceramid einschließt.

## Revendications

1. Accessoire pour stomie (10) et enceinte d'emballage (18), comprenant :
un accessoire pour stomie (10), l'accessoire pour stomie présentant des première et seconde surfaces (12, 14), l'accessoire pour stomie étant formé d'un matériau adhésif ;
une enceinte d'emballage (18) comprenant :
un corps comportant une base (20) et une paroi latérale verticale (22) solidaire de la base et contiguë à celle-ci et présentant un bord périphérique (24) opposé à la base, la base et la paroi latérale définissant une zone de stockage (28), la base et la paroi latérale étant formées d'un premier matériau ; et
un élément de couvercle (34),
dans lesquels l'accessoire pour stomie (10) est positionné dans la zone de stockage, avec la première surface (12) positionnée sur la base,
dans lesquels l'élément de couvercle (34) est positionné sur la paroi latérale verticale et en contact avec la seconde surface (14) de l'accessoire pour stomie, et
**caractérisés en ce que** l'élément de couvercle (34) est formé d'un second matériau, le second matériau présentant une affinité plus élevée pour l'accessoire pour stomie (10) que l'affinité du premier matériau pour l'accessoire pour stomie.

2. Accessoire pour stomie (10) et enceinte d'emballage (18) selon la revendication 1, dans lesquels l'accessoire pour stomie (10) reste fixé au couvercle (34) lorsque le couvercle est retiré de l'enceinte d'emballage.

3. Accessoire pour stomie (10) et enceinte d'emballage (18) selon la revendication 1, dans lesquels le couvercle (34) est scellé à la paroi latérale verticale (22).

4. Accessoire pour stomie (10) et enceinte d'emballage (18) selon la revendication 1, dans lesquels le corps est formé de polyéthylène (PE), de polyéthylène téréphtalate (PET), de polypropylène, de polyester, de polyimide, de polyoxyméthylène, d'organosiloxane ou de matériaux cellulosiques ou à base de papier, ou de combinaisons de ceux-ci.

5. Accessoire pour stomie (10) et enceinte d'emballage (18) selon la revendication 1, dans lesquels le couvercle (34) est formé de polyéthylène (PE), de polyéthylène téréphtalate (PET), de polypropylène, de polyester, de polyimide, de polyoxyméthylène, d'organosiloxane, ou de matériaux cellulosiques ou à base de papier, ou de combinaisons de ceux-ci.

6. Accessoire pour stomie (10) et enceinte d'emballage (18) selon la revendication 4, dans lesquels le couvercle (34) est formé de polyéthylène (PE), de polyéthylène téréphtalate (PET), de polypropylène, de polyester, de polyimide, de polyoxyméthylène, d'organosiloxane ou de matériaux cellulosiques ou à base de papier, ou de combinaisons de ceux-ci.

7. Accessoire pour stomie (10) et enceinte d'emballage (18) selon la revendication 1, dans lesquels l'accessoire pour stomie est un anneau de barrière (10).

8. Accessoire pour stomie (10) et enceinte d'emballage (18) selon la revendication 7, dans lesquels l'anneau de barrière (10) est formé d'un matériau hydrocolloïde.

9. Accessoire pour stomie (10) et enceinte d'emballage (18) selon la revendication 8, dans lesquels le matériau d'anneau de barrière comporte en outre du céramide.
